# EUROPEAN PATENT APPLICATION

(11) **EP 4 710 929 A1**
(43) Date of publication of application: **18.03.2026**
(21) Application number: 23936672.7
(22) Date of filing: 08.05.2023
(51) Int. Cl.: A61K 31/7004, A61K 31/7008, A61K 31/198, A61K 9/08, A61K 9/00, A61P 31/14, A61P 31/20, A61P 37/00

(54) **ANTIVIRAL INFUSION COMPOSITION**

(71) Applicant: Lee, Geon Moo, Gwangju 62070 (KR); Lee, In Seong, Gwangju 62070 (KR); Lee, Jun Seong, Gwangju 62070 (KR); Chung, Mi Boon, Seoul 06502 (KR)
(72) Inventor: Lee, Geon Moo, Gwangju 62070 (KR); Lee, In Seong, Gwangju 62070 (KR); Lee, Jun Seong, Gwangju 62070 (KR); Chung, Mi Boon, Seoul 06502 (KR)
(74) Representative: Louis Pöhlau Lohrentz
(86) International application number: PCT/KR2023/006231
(87) International publication number: WO 2024/232448

(57) **Abstract**

Disclosed is an antiviral infusion composition containing specific sugars.

## Description

### TECHNICAL FIELD

The present invention relates to an antiviral infusion composition.

### BACKGROUND ART

Key substances studied in the field of life sciences include proteins, amino acids, nucleic acids, fats, fatty acids, carbohydrates, polysaccharides, and sugars. Life science and medical researchers have long recognized proteins as the primary communication molecules within the body. However, they have concluded that proteins alone are insufficient for transmitting all messages into cells. Accordingly, they have begun studying glycoproteins, which are combinations of protein and carbohydrate molecules.

Eight types of sugars, including glucose (Glu), galactose (Gal), mannose (Man), L-fucose (Fuc), xylose (Xy), N-acetylglucosamine (GlcNAc), N-acetylgalactosamine (GalNAc), and N-acetylneuraminic acid (NANA; or sialic acid), play a critical role in maintaining physiological activity in the body.

The present inventors confirmed that an infusion composition containing the above-mentioned eight types of sugars has an antiviral effect, thereby completing the present invention.

### DETAILED DESCRIPTION OF THE INVENTION

### TECHNICAL PROBLEM

An object of the present invention is to provide an antiviral infusion composition containing a specific sugar.

### TECHNICAL SOLUTION

To achieve the above-described technical object, the present invention discloses an antiviral infusion composition containing a specific sugar.

Specifically, the infusion composition of the present invention contains sugars of glucose, galactose, mannose, fucose, xylose, N-acetylglucosamine, N-acetylgalactosamine and N-acetylneuraminic acid.

The infusion composition of the present invention has antiviral activity particularly against retrovirus, lentivirus, adenovirus, or the like.

The infusion composition of the present invention may treat, alleviate, or prevent diseases caused by viruses (e.g., acquired immunodeficiency syndrome, respiratory infection, digestive infection, otitis media, pharyngitis, laryngitis, keratitis, conjunctivitis, influenza, cold, skin infection, genital infection, hepatitis, etc.).

In addition, the present invention relates to an infusion composition for enhancing immunity against viruses, containing glucose, galactose, mannose, fucose, xylose, N-acetylglucosamine, N-acetylgalactosamine, and N-acetylneuraminic acid.

The infusion composition may further include one or more types of amino acid.

Here, as the amino acid, one or more 20 amino acids that may constitute proteins of the human body may be selected, and for example, the amino acid may be one or more selected from the group consisting of isoleucine, leucine, lysine acetate, methionine, phenylalanine, threonine, tryptophan, valine, alanine, arginine, aspartic acid, cysteine, glutamic acid, histidine, proline, serine, tyrosine, and glycine.

The infusion composition according to the present invention may be infused intravenously.

In addition, the present invention relates to a method for treating a disease caused by a virus or enhancing immunity against a virus by administering the above-described infusion composition to a human.

### ADVANTAGEOUS EFFECTS

The infusion composition according to the present invention exhibits excellent antiviral effects.

### DESCRIPTION OF THE DRAWINGS

FIG. 1 illustrates the results of an antiviral experiment of a composition of the present invention against a retrovirus.
FIG. 2 illustrates the results of an antiviral experiment of a composition of the present invention against a lentivirus.
FIG. 3 illustrates the results of an antiviral experiment of a composition of the present invention against an adenovirus.
FIG. 4 illustrates the results of an interferon expression effect experiment of a composition of the present invention.

### MODES OF THE INVENTION

The present invention will be described in detail with reference to the following examples. However, the following examples are intended only to illustrate the present invention and are not intended to limit the scope of the present invention.

### Example 1: Preparation of a sugar-containing infusion

Eight types of sugars (glucose, galactose, mannose, fucose, xylose, N-acetylglucosamine, N-acetylgalactosamine and N-acetylneuraminic acid) obtained from Sigma-Aldrich (St. Louis, Missouri, USA) were dissolved in water for injection (JW Pharmaceutical). Aspartic acid (L-aspartic acid), an amino acid, was used as a stabilizer.

A IN NaOH solution (Samchun Pure Chemical Co.) was used to adjust the pH of the prepared sugar-containing infusion. A pH meter (METTLER TOLEDO Seven Compact pH/Ion) was used to adjust the pH to 7.0.

Table 1 shows the composition of the sugar-containing infusion.

**[Table 1]**

| | Component | Sugar-containing infusion |
|---|---|---|
| Sugar (mg) | L-(-)-Fucose | 31 |
| | D-(+)-Galactose | 31 |
| | D-(+)-Xylose | 31 |
| | D-(+)-Glucose | 31 |
| | D-(+)-Mannose | 31 |
| | N-Acetylneuraminic acid | 31 |
| | N-Acetyl-D-galactosamine | 31 |
| | N-Acetyl-D-glucosamine | 31 |
| Stabilizer (mg) | Aspartic acid | 20 |
| Solvent | Water for injection | q.s. |
| pH adjustor (µl) | 1N NaOH | q.s. |
| Total | | 10mL |

| | | |
|---|---|---|
| * This is a composition table based on a total of 10 mL (actually, 50 mL was prepared). ** The content of the eight types of sugars in the above composition is 31 mg x 8 / 10 mL ≒ 2.5 (w/v)%. | | |

### Example 2: Experiment on retrovirus

### A. Cell plating

On Day 1, HEK293T cells cultured in Dulbecco's Modified Eagle Medium (DMEM) were plated in a 6-well plate at 5 × 10⁵ cells/well and cultured in an incubator (37 °C, CO₂ 5%) for 24 hours.

### B. Sugar mixture treatment

On Day 2, the sugar-containing composition prepared in Example 1 was added to the wells of the test group. Here, the total of the eight types of added sugars was diluted to a concentration of 0.25 (w/v)%, 0.5 (w/v)% and 1 (w/v)% based on the total amount of the composition present in each well (i.e., based on the total amount of the entire composition including the added sugar-containing composition).

No sugar-containing composition was added to the control wells.

Thereafter, the cells were cultured in an incubator (37 °C, CO₂ 5%) for 24 hours.

### C. Viral infection

On Day 3, green fluorescent protein (GFP)-tagged retrovirus or an empty vector (pMD2.G or psPAX2) which does not express the virus was added to wells and incubated for 48 hours to prepare an experimental group in which cells were infected with the virus and a control group in which cells were not infected with the virus, respectively.

### D. Verification of effects

On Day 5, the culture medium in each well was removed, and cells were washed twice with phosphate-buffered saline (PBS) buffer. Cells were then detached from the wells by trypsinization.

Thereafter, PBS buffer was added to each well and pipetted into e-tubes. The cells were centrifuged at 3,000 rpm for five minutes, and after removing the supernatant, suspended in fluorescence-activated cell sorting (FACS) buffer.

Thereafter, the number of virus-infected cells was measured using GFP-derived fluorescence.

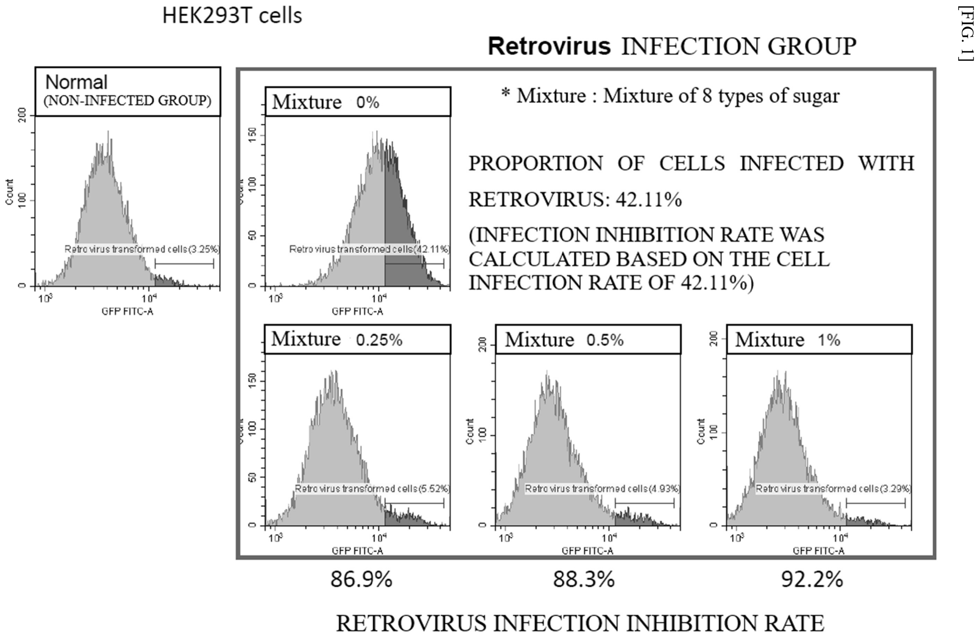

The experimental results are shown in FIG. 1. The experimental results showed that treatment with the sugar-containing composition of the present invention inhibited retrovirus infection in cells by more than 80%.

### Example 3: Experiment on lentivirus

### A. Cell plating

On Day 1, MDA-MB-231 cells cultured in DMEM were plated in a 6-well plate at a density of 5 × 10⁵ cells/well and cultured in an incubator (37 °C, CO₂ 5%) for 24 hours.

### B. Sugar mixture treatment

On Day 2, the sugar-containing composition prepared in Example 1 was added to the wells of the test group. Here, the total of the eight types of added sugars was diluted to a concentration of 1 (w/v)% based on the total amount of the composition present in each well (i.e., the total amount of the entire composition, including the added sugar-containing composition).

No sugar-containing composition was added to the control wells.

Thereafter, the cells were cultured in an incubator (37 °C, CO₂ 5%) for 24 hours.

### C. Viral infection

On Day 3, green fluorescent protein (GFP)-tagged lentivirus or an empty vector, which does not express the virus, was added to wells and incubated for 24 hours to prepare an experimental group in which cells were infected with the virus and a control group in which cells were not infected with the virus, respectively.

### D. Verification of effects

On Day 4, the culture medium in each well was removed, and cells were washed twice with PBS buffer. Cells were then detached from the wells by trypsinization.

Thereafter, PBS buffer was added to each well and pipetted into e-tubes. The cells were centrifuged at 3,000 rpm for five minutes, and after removing the supernatant, suspended in FACS buffer.

Thereafter, the number of virus-infected cells was measured using GFP-derived fluorescence.

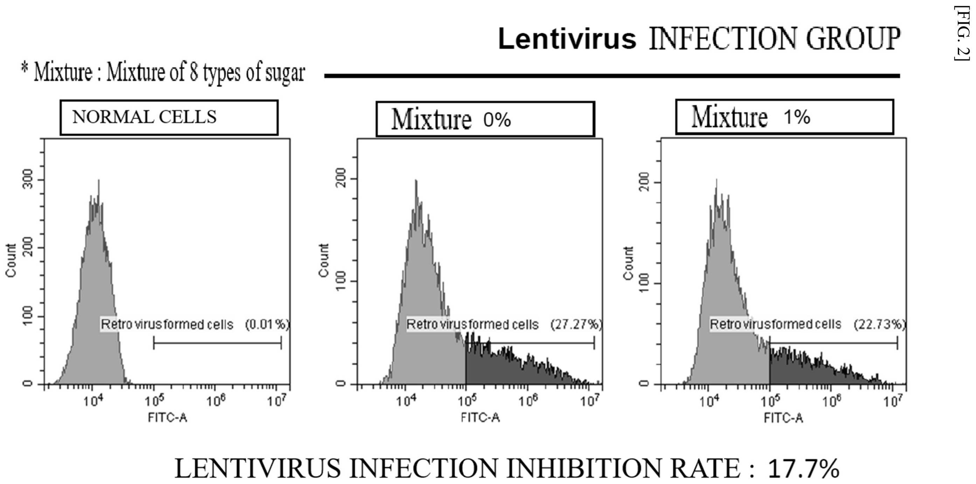

The experimental results are shown in FIG. 2. The experimental results showed that treatment with the sugar-containing composition of the present invention inhibited lentivirus infection in cells by about 17%, indicating that the composition has an excellent antiviral effect also on lentivirus.

### Example 4: Experiment on adenovirus

### A. Cell plating

On Day 1, HEK 293T cells cultured in DMEM were plated in a 6-well plate at a density of 5 × 10⁵ cells/well and cultured in an incubator (37 °C, CO₂ 5%) for 24 hours.

### B. Sugar mixture treatment

On Day 2, the sugar-containing composition prepared in Example 1 was added to the wells of the test group. Here, the total of the eight types of added sugars was diluted to a concentration of 1 (w/v)% based on the total amount of the composition present in each well (i.e., the total amount of the entire composition including the added sugar-containing composition).

No sugar-containing composition was added to the control wells.

Thereafter, the cells were cultured in an incubator (37 °C, CO₂ 5%) for 24 hours.

### C. Viral infection

On Day 3, green fluorescent protein (GFP)-tagged adenovirus or an empty vector, which does not express the virus, was added to wells and incubated for 24 hours to prepare an experimental group in which cells were infected with the virus and a control group in which cells were not infected with the virus, respectively.

### D. Verification of effects

On Day 4, the culture medium in each well was removed, and cells were washed twice with PBS buffer. Cells were then detached from the wells by trypsinization.

Thereafter, PBS buffer was added to each well and pipetted into e-tubes. The cells were centrifuged at 3,000 rpm for five minutes, and after removing the supernatant, suspended in FACS buffer.

Thereafter, the number of virus-infected cells was measured using GFP-derived fluorescence.

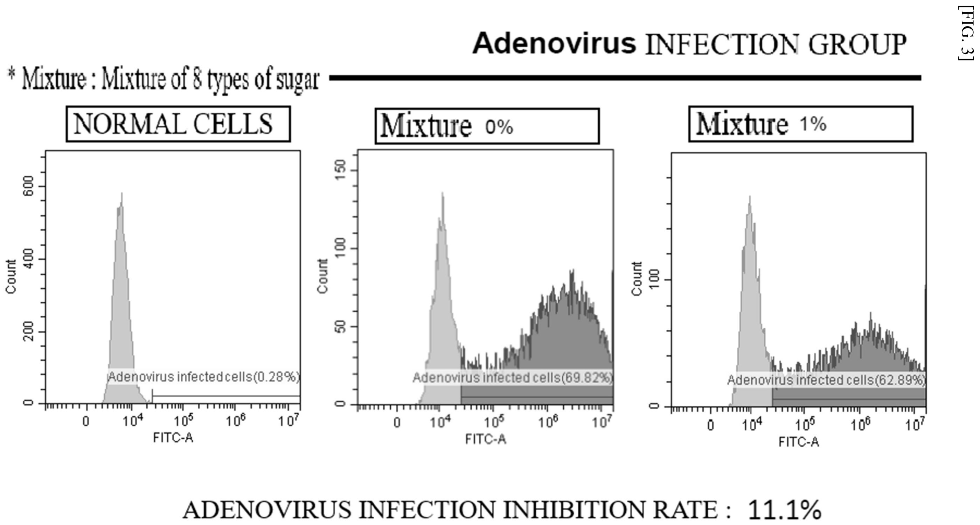

The experimental results are shown in FIG. 3. The experimental results showed that treatment with the sugar-containing composition of the present invention inhibited adenovirus infection in cells by about 11%, indicating that the composition has an excellent antiviral effect also on adenovirus.

### Example 5: Experiment on enhancing immunity against viruses

### A. Cell plating

On Day 1, RAW 264.7 cells cultured in DMEM were plated in a 24-well plate at a density of 1 × 10⁶ cells/well and cultured in an incubator (37 °C, CO₂ 5%) for 24 hours.

### B. Sugar mixture treatment

On Day 2, the sugar-containing composition prepared in Example 1 was added to the wells of the test group. Here, the total of the eight types of added sugars was diluted to a concentration of 1 (w/v)% based on the total amount of the composition present in each well (i.e., the total amount of the entire composition including the added sugar-containing composition). No sugar-containing composition was added to the control wells.

After 30 minutes of adding the mixture of eight types of sugars, the cells were treated with 200 µg/ml of Poly (I:C) and cultured in an incubator (37 °C, CO₂ 5%) for 24 hours.

### C. Verification of effects

On Day 3, cells were harvested, and RNA was collected from the cells.

After RNA collection, cDNA was synthesized, and real-time PCR was performed to measure mRNA expressing interferon α and interferon β.

The primer information for PCR is shown below.

**[Table 2]**

| | | |
|---|---|---|
| Interferon α | F | CAACACCTACACAGGTTACC |
| | R | AGTGGCTTCCCAGATGTTCC |
| Interferon β | F | TCCAAGAAAGGACGAACATT |
| | R | TGAGGACATCTCCCACGTCA |

The PCR conditions are shown below.

**[Table 3]**

| Stage | Step | Temperature (°C) | Time | Cycles |
|---|---|---|---|---|
| Hold stage | step 1 | 50 | 2 minutes | 1 |
| | step 2 | 96 | 10 minutes | |
| PCR stage | step 1 | 96 | 15 seconds | 40 |
| | step 2 | 60 | 1 minute | |
| Melt curve stage | step 1 | 96 | 15 seconds | 1 |
| | step 2 | 60 | 1 minute | |
| | step 3 (dissociation) | 96 | 15 seconds | |

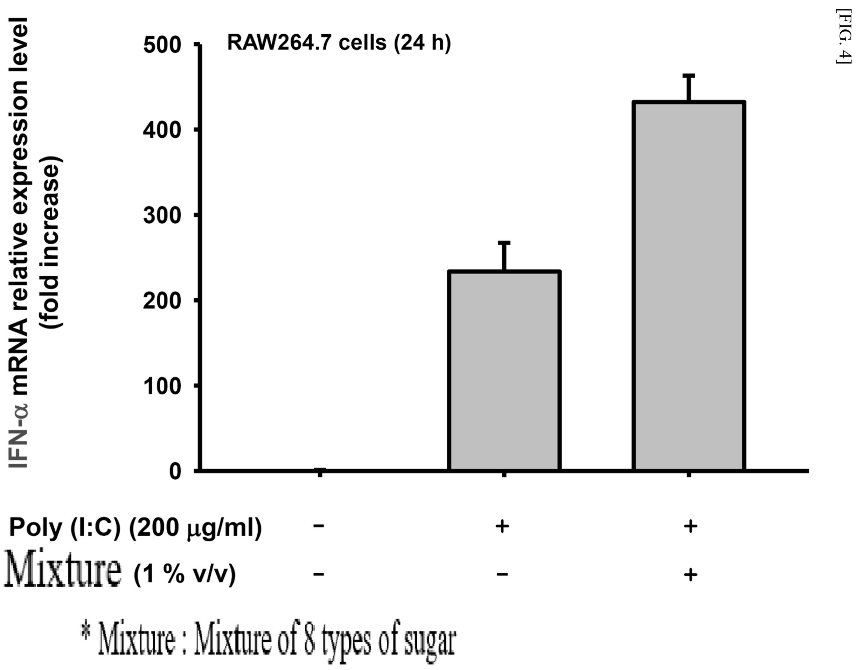

The experimental results are shown in FIG. 4. As a result of the experiment, it was confirmed that the composition according to the present invention increases the production of interferon (IFN) α, which is essential for viral immunity to fight viruses in the human body.

### INDUSTRIAL APPLICABILITY

The infusion composition of the present invention has excellent antiviral effects and immunity-enhancing effects against viruses.

## Claims

1. An antiviral infusion composition comprising glucose, galactose, mannose, fucose, xylose, N-acetylglucosamine, N-acetylgalactosamine and N-acetylneuraminic acid.

2. The antiviral infusion composition of claim 1, wherein the virus is a retrovirus, lentivirus or adenovirus.

3. The antiviral infusion composition of claim 1 or claim 2, further comprising one or more types of amino acid.

4. An infusion composition for enhancing immunity against viruses, comprising glucose, galactose, mannose, fucose, xylose, N-acetylglucosamine, N-acetylgalactosamine and N-acetylneuraminic acid.

5. The infusion composition of claim 1, further comprising one or more types of amino acid.
